# FASCICULE DE BREVET EUROPEEN

(11) **EP 3 972 548 B1**
(45) Date de publication et mention de la délivrance du brevet: **09.08.2023**
(21) Numéro de dépôt: 20727281.6
(22) Date de dépôt: 20.05.2020
(51) Int. Cl.: A61F 13/08

(54) **BANDE DE COMPRESSION COMPRENANT UNE COUCHE DE CAPITONAGE**
KOMPRESSIONSBAND MIT EINER POLSTERSCHICHT
COMPRESSION STRIP COMPRISING A PADDING LAYER

(30) Priorité: 22.05.2019 FR 1905366
(43) Date de publication de la demande: 30.03.2022
(73) Titulaire: THUASNE, 92300 Levallois-Perret (FR)
(72) Inventeur: GALLIEN, Nathalie, 42100 Saint-Etienne (FR); BREUIL, Laurent, 42240 Unieux (FR); AGUD, Adrien, 69007 Lyon (FR); CONVERT, Reynald, 69530 Brignais (FR)
(74) Mandataire: Lavoix
(86) Numéro de dépôt international: PCT/EP2020/064090
(87) Numéro de publication internationale: WO 2020/234362

(56) Documents cités:
- EP-A2- 0 878 179
- WO-A1-2013/001312
- US-A1- 2008 195 019
- US-A1- 2011 071 453

## Description

La présente invention concerne une bande de compression pour exercer une pression prédéfinie sur une partie de membre d'un patient, du type comprenant au moins une couche de compression destinée à appliquer la pression prédéfinie, ladite couche de compression étant élastique au moins selon sa direction longitudinale, correspondant à la longueur de la bande de compression, et au moins une couche de capitonnage non tissée, destinée à être appliquée contre la peau de la partie de membre, ladite couche de capitonnage étant fixée par entremêlement d'une partie des fibres de la couche de capitonnage dans la couche de compression.

L'invention concerne également un procédé de réalisation d'une telle bande de compression.

Les bandes de compression sont généralement utilisées dans le traitement de pathologies vasculaires, telles que les affections veineuses chroniques, les thromboses veineuses, les lymphoedèmes, afin de lutter contre la stase liquidienne. De telles bandes de compression peuvent également être utilisées en traumatologie ou orthopédie pour maintenir un membre dans certaines positions.

Pour appliquer une pression sur le membre, une bande de compression est enroulée autour de la partie du membre à traiter avec une certaine tension. La tension est choisie pour que la bande applique la pression nécessaire pour traiter la pathologie identifiée en fonction du module élastique de la bande et de la technique de pose de la bande.

L'efficacité du traitement par bande de compression réside dans l'application du niveau de pression adéquate pour la pathologie à traiter ainsi que dans l'observance du traitement faisant intervenir le prescripteur du traitement de compression, le pharmacien délivrant la bande de compression adéquate, le praticien ou la personne appliquant la bande de compression et le patient.

La pression que le dispositif exerce à la surface de la jambe dépend de la morphologie de la jambe, de la tension de la bande, de la technique de pose et de la capacité de la bande à tenir sur la jambe (voir « Experimental Investigation of Pressure Applied on the Lower Leg by Elastic Compression Bandage » (« Etude expérimentale de pression appliquée sur la partie inférieure de la jambe par une bande de compression élastique ») de F. Chassagne, F. Martin, P. Badel, R. Convert, R. Giraux et J. Molimard dans Ann Biomed Eng. Dec2015.43(12):2967-77). Plus il y a de couches de bandes superposées, moins la pression exercée sera maitrisée (voir « Superimposition of elastic and nonelastic compression bandages » (« superposition de bandes élastique et non élastique ») de F. Chassagne, C. Helouin-Desenne, J. Molimard, R. Convert, P. Badel, P. Giraux dans Journal of Vascular Surgery : Venous and Lymphatic Disorders. Nov 2017;5(6):851-8), et moins la pose sera reproductible. Plus il y a de couche de bandes, plus le dispositif sera épais, et moins il sera confortable pour le patient (sensation de chaleur, difficulté pour transférer l'humidité, etc.).

Il est également important que la pression exercée par le dispositif de compression soit maintenue dans le temps. C'est le cas quand le dispositif a un comportement élastique (voir « Classification of compression bandages » (« Classification de bandes de compression ») de E. Dréan, R. Convert, L. Schacher dans The Fiber Society Fall's 2018 Technical Meeting and Conférence ; Davis ; Californie Etats-Unis ; 2018).

Afin de protéger la partie de membre, par exemple lorsque la peau du patient est fragilisée, ou afin de combler les reliefs dans la partie de membre, par exemple les reliefs naturels, tels que les malléoles, ou les reliefs dus à certaines dysmorphies, de manière à répartir la pression sur la zone, une bande de capitonnage peut être d'abord appliquée autour de la partie de membre, la bande de compression étant alors appliquée sur la bande de capitonnage pour comprimer la partie de membre et la bande de capitonnage. Une telle bande de capitonnage est par exemple formée par un non-tissé.

Pour faciliter la pose de la bande de capitonnage et de la bande textile, il a été proposé d'assembler ces bandes ensemble avant la pose afin de n'avoir qu'une seule opération de pose à effectuer, c'est-à-dire de ne pas avoir à poser la bande de capitonnage puis la bande textile en deux étapes de pose successives. En outre, la reproductibilité de la pose est également améliorée par rapport à des systèmes comprenant plusieurs bandes distinctes. Un tel assemblage est décrit dans WO2013/001312 A1 par exemple.

Cependant, la pose reste une étape délicate au cours de laquelle la bande de compression doit être soigneusement conformée à la morphologie de la partie de membre et ne pas former de plis. En effet, une pose mal effectuée réduit l'efficacité du traitement, voir empire la situation du patient, et peut générer une gêne forte pour le patient.

L'un des buts de l'invention est de pallier ces inconvénients en proposant une bande de compression permettant de réaliser la fonction de compression et celle de capitonnage tout en étant simple à poser et apportant un confort accru au patient.

A cet effet, l'invention concerne une bande de compression du type précité, dans laquelle la bande de compression est en outre élastique selon sa direction transversale, correspondant à la largeur de la bande de compression.

En prévoyant une bande de compression élastique dans la direction longitudinale et dans la direction transversale, c'est-à-dire une bande de compression bi-élastique dans son ensemble, la pose de la bande de compression est facilitée car celle-ci s'adapte naturellement à la morphologie de la partie de membre et car elle n'est pas susceptible de former des plis. Ainsi, le confort du patient est amélioré et le traitement plus facile à observer.

Selon d'autres caractéristiques de la bande de compression selon l'invention, prises isolément ou selon toute combinaison techniquement envisageable :
- la bande de compression comprend une couche de mousse s'étendant entre la couche de compression et la couche de capitonnage, ladite couche de mousse étant fixée à la couche de compression et à la couche de capitonnage par entremêlement d'une partie des fibres de la couche de capitonnage dans la couche de compression et dans la couche de mousse ;
- la couche de mousse présente une épaisseur, mesurée selon une direction perpendiculaire à la direction longitudinale et à la direction transversale, sensiblement comprise entre 1 mm et 3,5 mm, mesurée suivant la norme NF EN ISO 5084 :2016, sous une pression de 0,5kPa ;
- la couche de compression présente une extensibilité d'au moins 30% selon la direction longitudinale ;
- la couche de compression présente une extensibilité selon sa direction longitudinale sensiblement comprise entre 50% et 120% ;
- la couche de compression présente une extensibilité d'au moins 10% selon la direction transversale ;
- la bande de compression présente une masse surfacique sensiblement comprise entre 200 g/m² et 800 g/m².

L'invention concerne également un procédé de réalisation d'une bande de compression telle que décrite ci-dessus, comprenant les étapes suivantes :
- réaliser un tissu formant une couche de compression, ledit tissu étant élastique selon sa direction longitudinale et selon sa direction transversale,
- étirer la couche de compression au moins selon sa direction longitudinale ;
- disposer des fibres sur la couche de compression étirée, ou pré-aiguilleter une nappe de fibres et la disposer sur la couche de compression étirée ;
- réaliser une couche de capitonnage et fixer ladite couche de capitonnage sur la couche de compression étirée par entremêlement des fibres entre elles et par entremêlement d'une partie des fibres dans la couche de compression.

Selon d'autres caractéristiques du procédé de réalisation selon l'invention, prises isolément ou selon toute combinaison techniquement envisageable :
- le procédé comprend une étape de disposition d'au moins une couche de mousse sur la couche de compression étirée, les fibres étant disposées sur la couche de mousse, la couche de capitonnage étant réalisée et fixée à la couche de mousse et à la couche de compression par entremêlement des fibres entre elles et par entremêlement d'une partie des fibres dans la couche de mousse et dans la couche de compression ;
- l'étape d'entremêlement des fibres est une étape d'aiguilletage ou d'entremêlement par jet d'air ou jet d'eau.

D'autres aspects et avantages de l'invention apparaîtront à la lecture de la description qui suit, donnée à titre d'exemple et faite en référence aux dessins annexés, dans lesquels :
- [Fig 1] la Fig. 1 est une représentation schématique en coupe d'une bande de compression selon un premier mode de réalisation de l'invention,
- [Fig 2] la Fig. 2 est une représentation schématique en coupe d'une bande de compression selon un deuxième mode de réalisation de l'invention,
- [Fig 3] la Fig. 3 est une représentation schématique d'un exemple de structure d'une couche de compression qui peut être utilisée dans une bande de compression selon l'invention, et
- [Fig 4] la Fig. 4 est une représentation schématique en coupe d'une étape du procédé de réalisation d'une bande de compression selon le deuxième mode de réalisation de l'invention.

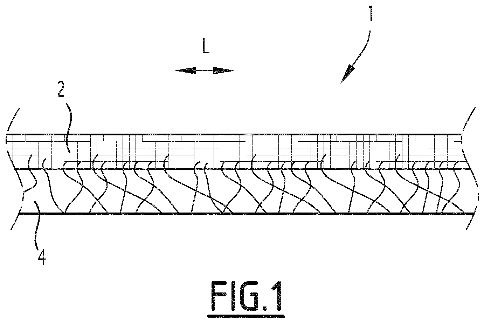

En référence à la Fig. 1, on décrit une bande de compression 1 comprenant, selon un premier mode de réalisation, une couche de compression 2 et une couche de capitonnage 4 assemblées entre elles.

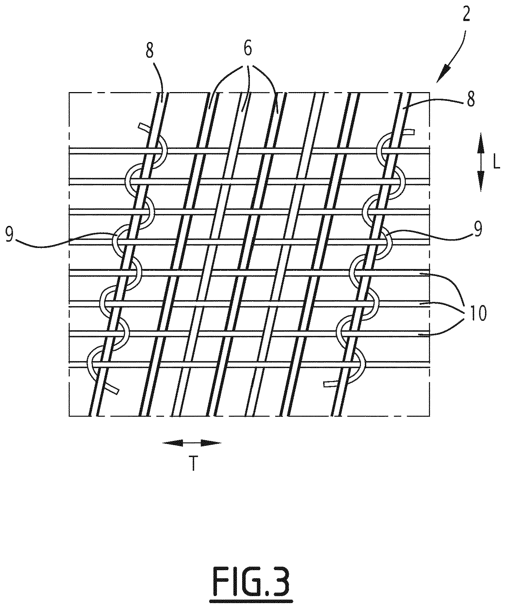

La couche de compression 2 est par exemple formée par un textile tissé, comme représenté sur la Fig. 3. La couche de compression 2 est ainsi formée par une bande de tissu s'étendant principalement selon une direction longitudinale L, selon laquelle la longueur de la couche de compression 2 est mesurée, et selon une direction transversale T, selon laquelle la largeur de la bande de compression 2 est mesurée, la direction transversale T étant sensiblement perpendiculaire à la direction longitudinale L.

La couche de compression 2 présente une élasticité dans le sens de la direction longitudinale L et dans le sens de la direction transversale T. Par élasticité, on entend que la couche de compression 2 peut être étirée selon la direction longitudinale et/ou selon la direction transversale de sorte à en augmenter la longueur et/ou la largeur et qu'elle retrouve sa longueur et/ou sa largeur initiale en l'absence d'effort tendant à l'étirer. Ainsi, la couche de compression 2 est bi-élastique, ce qui signifie qu'elle peut être étirée de façon réversible dans la direction de sa longueur et dans la direction de sa largeur. Selon un mode de réalisation, l'extensibilité de la couche de compression 2 selon la direction longitudinale L est d'au moins 30%. C'est-à-dire que la longueur de la couche de compression 2 peut augmenter d'au moins 30% lorsque la couche de compression est étirée selon la direction longitudinale L. Selon un mode de réalisation particulier, l'extensibilité maximale de la couche de compression 2 selon la direction longitudinale est sensiblement comprise entre 50% et 120%. Selon un mode de réalisation, l'extensibilité de la couche de compression 2 selon la direction transversale T est d'au moins 10%. C'est-à-dire que la largeur de la couche de compression 2 peut augmenter d'au moins 10% lorsque la couche de compression est étirée selon la direction transversale T. Selon un mode de réalisation particulier, l'extensibilité de la couche de compression 2 selon la direction transversale est sensiblement comprise entre 10% et 70%.

Selon un mode de réalisation représenté sur la Fig. 3, la couche de compression 2 est un tissu comprenant des premiers fils de chaîne 6, des deuxièmes fils de chaîne 8, des troisièmes fils de chaine 9, et des fils de trame 10.

Les premiers fils de chaîne 6 s'étendent selon la direction longitudinale parallèlement les uns aux autres. Selon un mode de réalisation, les premiers fils de chaîne 6 sont des fils élastiques. Selon un autre mode de réalisation, les fils de chaîne 6 ne sont pas élastiques. Selon un mode de réalisation, les premiers fils de chaîne 6 sont des fils par exemple à multifilaments en matière textile thermoplastique, texturés mécaniquement et thermiquement. De tels fils permettent de suivre l'allongement et le retrait du tissu sans créer de défaut du fait de leur aptitude à se comporter comme des ressorts. En outre, lorsque la bande de compression 1 est réalisée par aiguilletage, comme cela sera décrit ultérieurement, cette opération ne provoque pas de défaut visuel dans l'aspect de la bande car la structure souple des fils permet à l'aiguille de traverser le tissu sans déplacer les fils. Les premiers fils de chaîne 6 sont par exemple réalisé en polyamide, polyester ou polypropylène. Selon divers exemples, les premiers fils de chaîne 6 sont par exemple des fils en élasthanne nus, des fils guipés avec une âme en élasthanne, des fils assemblés par jet d'air, etc. Les premiers fils de chaîne 6 permettent ainsi un allongement élastique de la couche de compression 2 selon la direction longitudinale.

Les deuxièmes fils de chaîne 8 s'étendent également selon la direction longitudinale et sont insérés dans le tissu entre certains des premiers fils de chaîne 6. Ainsi, selon un exemple, le tissu comprend une alternance de cinq premiers fils de chaîne 6, d'un deuxième fil de chaîne 8 et d'un troisième fil de chaîne 9 tissé en pas-de-gaze, comme représenté sur la Fig. 3. Les deuxièmes fils de chaîne 8 sont des fils élastiques et présentent une élasticité sensiblement supérieure à celle des premiers fils de chaîne 6. Selon un mode de réalisation, les deuxièmes fils de chaîne 8 sont des fils d'élasthanne ou contenant de l'élasthanne. Ces fils présentent par exemple un titrage compris entre 140 et 500 décitex, par exemple 390 décitex. Selon un mode de réalisation, les deuxièmes fils de chaîne 8 sont en outre des fils guipés, le guipage consistant à enrouler au moins un fil de couverture autour d'un fil d'âme souvent en élasthanne. Selon un autre mode de réalisation, les deuxièmes fils de chaine 8 sont des fils assemblés air, composés d'une âme élastique assemblée par jet d'air à un fil de couverture à multifilaments. Le fil de couverture est par exemple un fil de polyester, par exemple d'un titrage de 167 dtex. Selon un mode de réalisation particulier, les deuxièmes fils de chaîne 8 sont des fils double guipés, c'est-à-dire comprenant un fil d'âme et deux fils de guipage enroulés autour du fil d'âme. Les deuxièmes fils de chaîne 8 permettent de conférer ses propriétés de compression à la couche de compression 2 lorsque la bande de compression est enroulée autour d'une partie d'un membre en étant étirée selon la direction longitudinale L. Ces fils de chaine 8 augmentent également la capacité d'allongement longitudinal de la bande. Le troisième fil de chaîne 9 tissé en pas-de-gaze est par exemple de même composition et de même nature que les deuxièmes fils de chaîne 8. Ils ont pour fonction d'améliorer la structure et la stabilité du tissu et de conférer au tissu une élasticité selon la direction transversale T, sans nécessiter que les fils de trame 10 soient élastique.

Les fils de trame 10 s'étendent selon la direction transversale T parallèlement les uns aux autres et sont tissés avec les premiers, deuxièmes et troisièmes fils de chaîne 6, 8 et 9. Comme indiqué précédemment, les fils de trame 10 peuvent ne pas être élastiques. Pour les mêmes raisons que pour les premiers fils de chaîne 6, les fils de trame sont par exemple des fils multifilaments thermoplastiques texturés présentant une souplesse et une douceur satisfaisantes, permettant de suivre le retrait du tissu et d'accepter le passage des aiguilles lorsque la bande est réalisée par aiguilletage. Selon un mode de réalisation, les fils de trame 10 sont des fils de polyamide. Selon un mode de réalisation où les fils de trame 10 sont élastiques, ils peuvent permettre ainsi un allongement élastique de la couche de compression 2 selon la direction transversale.

La couche de compression 2 formée par un tissu tel que décrit ci-dessus présente ainsi une masse surfacique sensiblement comprise entre 150 g/m² et 400 g/m², par exemple entre 200 et 300 g/m², un allongement selon la direction longitudinale compris entre 30% et 120% et un allongement selon la direction transversale supérieur à 10%.

La couche de capitonnage 4 est formée par un non-tissé formé par un mélange de fibres entremêlées entre elles. Le non-tissé est par exemple formé par un mélange de fibres de viscose et de polyester d'une masse surfacique sensiblement comprise entre 60g/m² et 200 g/m², par exemple environ 95 g/m² lorsque la bande de compression ne comprend pas de couche de mousse, et environ 120 g/m² lorsque la bande de compression comprend une couche de mousse, comme cela sera décrit ultérieurement. La couche de capitonnage 4 présente par exemple une épaisseur sensiblement comprise entre 0.5 mm et 3.5 mm. L'épaisseur est mesurée selon une direction perpendiculaire à la direction longitudinale L et à la direction transversale T. Une telle couche de capitonnage 4 est destinée à être appliquée contre la peau de la partie de membre d'un patient lorsque la bande de compression 1 est enroulée autour de cette partie de membre. La couche de capitonnage 4 permet de protéger la peau et éventuellement de combler les reliefs dans la partie de membre dus à certaines dysmorphies. La couche de capitonnage 4 améliore également la fonction de compression de la bande de compression en uniformisant la pression exercée par la couche de compression 2, cette pression n'étant en effet plus principalement concentrée sur les fils de chaine 8 ou sur les zones à faible rayon de courbure comme les malléoles ou le tibia par exemple.

L'assemblage de la couche de capitonnage 4 et de la couche de compression 2 se fait par entremêlement d'une partie des fibres de la couche de capitonnage 4 dans la couche de compression 2. En d'autres termes, certaines des fibres de la couche de capitonnage 4 sont « accrochées » dans le tissu formant la couche de compression 2. L'étape d'entremêlement se fait sur la couche de compression 2 alors que celle-ci est étirée au moins selon la direction longitudinale L. C'est-à-dire que, pendant l'entremêlement, la couche de compression 2 est maintenue étirée selon la direction longitudinale de sorte à présenter une longueur supérieure à celle que la couche de compression 2 présente en l'absence d'effort tendant à étirer la couche de compression 2. Cela permet de conférer à l'ensemble de la bande de compression 1, formée de l'assemblage de la couche de compression 2 et de la couche de capitonnage 4, le caractère bi-élastique de la couche de compression 2. Selon un mode de réalisation, la couche de compression est en outre étirée selon la direction transversale T, ce qui permet de conférer un plus grand allongement transversal à la bande de compression obtenue. Selon un mode de réalisation, l'étape d'entremêlement est réalisée alors que la couche de compression 2 est étirée à son maximum, c'est-à-dire à sa limite d'allongement, selon la direction longitudinale et selon la direction transversale.

L'entremêlement est par exemple réalisé par aiguilletage, par jet d'air ou par jet d'eau, comme cela sera décrit plus en détail ultérieurement. Selon un mode de réalisation, l'entremêlement d'une partie des fibres dans la couche de compression 2 se fait en même temps que l'entremêlement des fibres pour réaliser le non-tissé formant la couche de capitonnage 4, comme cela sera décrit plus en détail ultérieurement. En d'autres termes, selon ce mode de réalisation, la couche de capitonnage 4 est réalisée en même temps qu'elle est fixée à la couche de compression 2. Selon un mode de réalisation, la nappe de fibres formant la couche de capitonnage subit un premier aiguilletage visant à la solidifier légèrement avant d'être complètement constituée lors de son assemblage simultané avec la couche de compression 2.

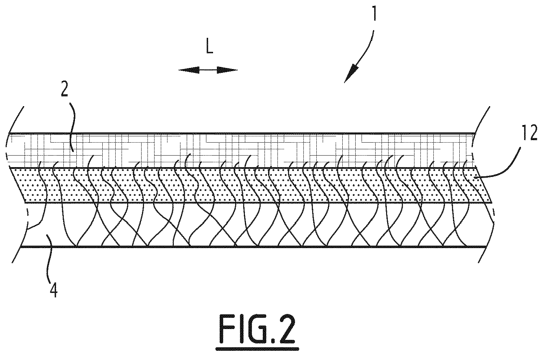

Un deuxième mode de réalisation de la bande de compression 1 selon l'invention et représenté sur la Fig. 2 va à présent être décrit.

Le deuxième mode de réalisation diffère du premier mode de réalisation par le fait que la bande de compression comprend en outre au moins une couche de mousse 12 s'étendant entre la couche de compression 2 et la couche de capitonnage 4 et fixée à ces couches afin d'améliorer l'effet de capitonnage de la bande de compression.

La couche de mousse 12 présente par exemple une épaisseur comprise entre 1 mm et 3,5 mm, cette épaisseur correspondant à la distance séparant la couche de compression 2 de la couche de capitonnage 4. L'épaisseur de la couche de mousse est mesurée selon la norme NF EN ISO 5084 :2016, sous une pression de 0,5kPa. La mousse est par exemple une mousse de polyuréthane. La couche de mousse 12 présente par exemple une densité sensiblement comprise entre 15 kg/m³ et 45 kg/m³. Une telle couche de mousse 12 permet d'améliorer la fonction de capitonnage de la bande de compression 1 par une augmentation de l'épaisseur entre la peau du patient et la bande de compression sans augmenter la masse surfacique de la couche de capitonnage 4. En outre, contrairement à une telle augmentation de la masse surfacique de la couche de capitonnage 4, la couche de mousse 12 apporte une force de résilience qui permet de maintenir l'épaisseur dans le temps et donc de prolonger l'effet de capitonnage dans le temps. La couche de mousse 12 améliore également la fonction de compression de la bande de compression en uniformisant la pression exercée par la couche de compression 2, cette pression n'étant en effet plus principalement concentrée sur les fils de chaine 8 ou sur les zones à faible rayon de courbure comme les malléoles ou le tibia par exemple.

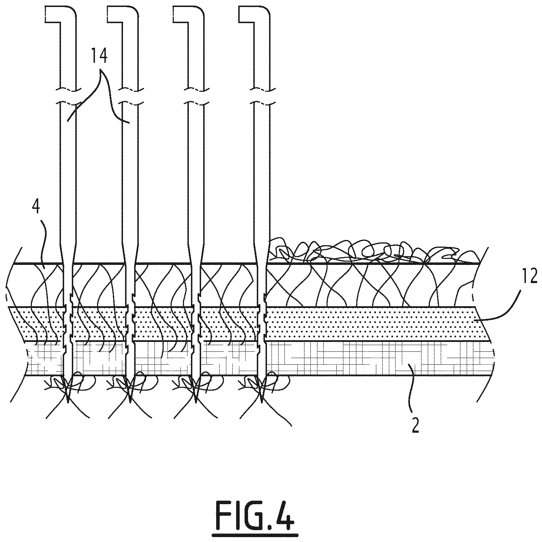

La couche de mousse 12 est fixée à la couche de compression 2 et à la couche de capitonnage 4 par entremêlement d'une partie des fibres de la couche de capitonnage 4 dans la couche de mousse 12 et dans la couche de compression 2, comme plus particulièrement représenté sur la Fig. 4. Ainsi, l'étape d'entremêlement est agencée pour qu'une partie des fibres de la couche de capitonnage 4 traverse la couche de mousse 12 et s'accroche dans le tissu formant la couche de compression 2.

Selon un mode de réalisation, deux couches de mousse 12 telles que décrites ci-dessus sont disposées entre la couche de compression 2 et la couche de capitonnage 4.

Le procédé de réalisation de la bande de compression 1 selon le deuxième mode de réalisation va à présent être décrit. Il convient de noter que le procédé de réalisation de la bande de compression 1 selon le premier mode de réalisation est identique à ce qui va être à présent décrit en omettant la couche de mousse 12.

Après réalisation de la couche de compression 2 par tissage, celle-ci est étirée dans la direction longitudinale et, selon un mode de réalisation, dans la direction transversale. Comme indiqué précédemment, cet étirement se fait par exemple jusqu'au maximum d'allongement de la bande compression selon ces deux directions. Cependant, il est entendu que l'allongement pourrait être fait uniquement selon la direction longitudinale et pas nécessairement jusqu'au maximum.

La couche de mousse 12 est ensuite déposée sur la couche de compression 2 étirée et les fibres destinées à former la couche de capitonnage 4 sont déposées sur la couche de mousse 12. Selon un mode de réalisation, la couche de mousse 12 n'est pas étirée avec la couche de compression 2. Selon un autre mode de réalisation, la couche de mousse 12 est étirée selon la direction longitudinale et/ou selon la direction transversale.

Ensuite, au cours d'une étape d'entremêlement, les fibres déposées sont entremêlées entre elles et une partie d'entre elles est entremêlée dans la couche de mousse 12 et dans la couche de compression 2 de sorte à réaliser la couche de capitonnage 4 et à la fixer simultanément à la couche de mousse 12 et à la couche de compression 2, comme représenté sur la Fig. 4. Sur cette figure, l'étape d'entremêlement est une étape d'aiguilletage, consistant, comme connu en soi, à faire passer des aiguilles 14 au travers des fibres déposées sur la couche de mousse 12, au travers de la couche de mousse 12 et jusqu'à la couche de compression 2 afin de réaliser en même temps la couche de capitonnage 4 et sa fixation sur la couche de mousse 12 et sur la couche de compression 2 étirée. En alternative d'un aiguilletage, l'étape d'entremêlement pourrait être réalisée par jet d'eau ou d'air, comme également connu en soi. Comme indiqué précédemment, le procédé peut comprendre une étape de pré-aiguilletage de la nappe de fibres afin de préformer la couche de capitonnage en lui donnant de la cohésion avant qu'elle soit complètement finalisée lors de son assemblage avec la couche de mousse 12 et la couche de compression 2. Ce pré-aiguilletage permet entre autres de pouvoir introduire dans l'aiguilleteuse la couche de compression 2 au-dessus de la nappe de fibre, facilitant la gestion de l'alimentation de la couche de compression 2 et de ses tensions.

Une fois que la couche de capitonnage 4 a été réalisée et fixée sur la couche de mousse 12 et la couche de compression 2, la couche de compression 2 est libérée des tensions de sorte à ce qu'elle retrouve sa forme initiale. On notera que la présence de la couche de mousse 12 permet, lors de la libération de la couche de compression 2, d'entraîner un enroulement de la bande de compression 1 sur elle-même dans le sens de la longueur, ce qui permet ensuite un stockage simplifié de la bande de compression et une pose également simplifiée sur un patient.

En variante du procédé décrit ci-dessus, le non-tissé de la couche de capitonnage 4 est réalisé avant d'être fixé sur la couche de mousse 12 et la couche de compression 2. Cependant, un tel procédé ajoute une étape supplémentaire de fixation de la couche de capitonnage 4 sur le reste de la bande de compression 1.

Dans le premier mode de réalisation, les fibres ou la couche de capitonnage 4 sont déposées directement sur la couche de compression 2 et fixées sur celle-ci.

On notera que plusieurs bandes de compression 1 peuvent être réalisées en même temps, en prévoyant une couche de compression 2 de grande largeur et en entremêlant les fibres de la couche de capitonnage 4 sur toute la largeur de la couche de compression 2. La bande de compression de grandes dimensions ainsi obtenues est ensuite découpée dans le sens de sa longueur pour former plusieurs bandes de compression, présentant chacune une largeur par exemple comprise entre 8 cm et 10 cm.

La bande de compression 1 ainsi obtenue présente une masse surfacique sensiblement comprise entre 200 g/m² et 800 g/m².

Comme indiqué précédemment, la bande de compression 1 selon l'invention, aussi bien dans son premier mode de réalisation que dans son deuxième mode de réalisation, est bi-élastique, ce qui en facilite la pose car elle s'adapte mieux à la morphologie du patient et ne forme pas de plis. A titre d'exemple, lorsque la bande de compression 1 est utilisée sur la jambe, le pied et l'articulation de la cheville d'un patient, ceux-ci présentent des morphologies complexes avec des courbures tridimensionnelles dans les plans frontal, transversal et sagittal. L'allongement longitudinal peut suffire à couvrir des variations de circonférence de la jambe pour passer de la cheville au mollet mais sans allongement transversal il est alors nécessaire de lisser à la main les plis qui se forment sur le segment cheville-mollet. En outre, plus la variation de circonférence entre la cheville et le mollet est grande et/ou plus la bande de compression est large, plus la pose s'avère délicate lorsque la bande n'est élastique que selon la direction longitudinale. La bande de compression bi-élastique de l'invention permet d'éviter la formation de ces plis et donc d'avoir à lisser la bande de compression lors de la pose même pour des bandes de compression particulièrement large et/ou pour des variations de circonférence importantes. La bande de compression bi-élastique permet notamment de couvrir le talon sans former de plis au niveau du talon d'Achille, sous le talon ou encore à la pliure entre le tibia et le pied, ce qui n'est pas possible avec une bande ne présentant qu'une élasticité selon la direction longitudinale.

Un exemple de bande de compression 1 présentant les caractéristiques décrites précédemment et réalisée selon le procédé décrit ci-dessus va à présent être décrit.

La couche de compression 2 est formée par un tissu armuré conforme à celui qui a été décrit en référence à la Fig. 3 et comprend des premiers fils de chaîne 6 de polyamide texturés par fausse-torsion 2/78 dtex, les deuxièmes et les troisièmes fils de chaîne 8 et 9 sont des fils double guipés de composition identique avec une âme en élasthanne 390 dtex et des couvertures de polyester 167 dtex. Les fils de trame 10 sont des fils de polyamide 4/78/34 (un fil composé de 4 bouts de 78 dtex et comprenant 34 filaments chacun), texturé en fausse torsion.

Le tissu, une fois réalisé, subit une opération d'ennoblissement, comprenant des étapes de vaporisage, d'imprégnation dans de l'eau et de séchage sur des rouleaux chauffés, afin de conférer au tissu ses propriétés mécaniques définitives ainsi que sa stabilité dimensionnelle.

Un tel tissu est bi-élastique et permet une accroche solide de la couche de capitonnage 4 par aiguilletage sans détérioration du tissu.

La couche de capitonnage 4 est formée par un non-tissé présentant une masse surfacique de 95 g/m² constitué à 30% de fibres de PES et 70% de fibres de viscose. Un tel non-tissé présente de bonnes caractéristiques de protection et de tolérance cutanées, d'absorption d'humidité pour éviter la macération et présente une longueur de fibres suffisante pour garantir une bonne accroche avec le tissu lors de l'aiguilletage.

En sortie de cardeuse, la nappe de fibres destinée à former la couche de capitonnage 4 subit un pré-aiguilletage, consistant en un aiguilletage à faible densité, afin de lui conférer une cohésion.

La nappe de non-tissé est ensuite simultanément définitivement constituée et fixée à la couche de compression 2 en étant aiguilletée sur le tissu décrit précédemment avec une densité d'aiguilletage comprise en 50 p/cm² et 100 p/cm² (points par centimètre carré). La précision de densité d'aiguilletage, dans cette plage, n'a que peu d'influence sur la bande de compression obtenue.

Une telle bande de compression 2 présente un allongement maximal compris entre 50% et 60%.

A titre d'exemple, pour traiter un ulcère veineux sur une cheville présentant une circonférence comprise entre 20 cm et 25 cm, ce qui correspond à la circonférence moyenne des femmes et des hommes de plus de 70 ans, les plus susceptibles d'être touchés par un tel ulcère, une pression comprise entre 30 mmHg et 40 mmHg doit être appliquée sur la cheville (d'après les recommandations de la Haute Autorité de Santé), ce qui correspond à une force exercée sur la cheville comprise entre 127 cN/cm et 212 cN/cm selon la loi de Laplace. Une telle force correspond à un allongement de cette bande de compression posée à double recouvrement compris entre 33% et 41, 5%, ce qui est déterminé par lecture de la courbe de sa force appliquée en fonction de son allongement.

Ainsi, la bande de compression selon l'exemple décrit ci-dessus permet de traiter l'ulcère veineux pour la majorité des chevilles des personnes prédisposées à contracter un ulcère veineux. En outre, la bande de compression assure une protection de la peau et une capacité à évacuer l'humidité. En outre, comme indiqué précédemment, la bande de compression est formée d'une bande unique, ce qui facilite la pose et en améliore la reproductibilité en comparaison avec des bandes de compression comprenant plusieurs bandes.

Selon un autre exemple, une couche de mousse 12 est ajoutée à la bande de compression décrite ci-dessus entre la couche de compression 2 et la couche de capitonnage 4, ce qui réduit d'avantage l'allongement maximal de la bande de compression. La mousse est une mousse de polyuréthane à base de polyester de 1.5 mm d'épaisseur et une densité de 32 g/m². La mousse possède un allongement supérieur à 200% pour ne pas limiter l'allongement de la bande au cours de la pose ou se déchirer à la suite de l'étirage de la bande de compression. La mousse présente en outre une résistance à la compression de 4 kPa à 40%. La bande de compression comprenant une telle couche de mousse présente un allongement maximal inférieur d'environ 5% par rapport à la bande compression sans couche de mousse, soit un allongement maximal d'environ 50%.

Selon encore un autre exemple, la couche de mousse 12 présente la même composition mais présente une épaisseur de 3 mm et une densité de 64 g/m², ce qui diminue encore l'allongement maximal de la bande de compression à environ 45%. La plage d'étirage d'utilisation est alors sensiblement comprise entre 22.75% et 30.2% d'allongement. Une couche de mousse d'une telle épaisseur augmente l'effet de capitonnage mais complexifie la pose de la bande de compression en raison d'une épaisseur de bande enroulée importante.

Le tableau ci-dessous résume les caractéristiques des bandes de compression selon les exemples décrits ci-dessus en comparaison avec une bande de compression formée uniquement d'une couche de compression.

**[Tableau 1]**

| | Masse surfacique (g/m²) | Allongeme nt maximal (%) | Plage de traitement de l'ulcère à prédominance veineuse | | | |
|---|---|---|---|---|---|---|
| | | | Force min (cN/cm) | Allongement min (%) | Force max (cN/cm) | Allongement max (%) |
| Tissu seul | 277 | 94,5 | 127 | 59,7 | 212 | 73,6 |
| Tissu + non-tissé | 392 | 55 | | 33 | | 41,5 |
| Tissu + mousse 1,5mm + non-tissé | 433 | 50 | | 27,7 | | 35,3 |
| Tissu + mousse 3mm + non-tissé | 509 | 45 | | 22,75 | | 30,2 |

## Revendications

1. Bande de compression (1) pour exercer une pression prédéfinie sur une partie de membre d'un patient, ladite bande de compression comprenant au moins une couche de compression (2) destinée à appliquer la pression prédéfinie, ladite couche de compression étant élastique au moins selon sa direction longitudinale (L), correspondant à la longueur de la bande de compression, et au moins une couche de capitonnage (4) non tissée, destinée à être appliquée contre la peau de la partie de membre, ladite couche de capitonnage (4) étant fixée par entremêlement d'une partie des fibres de la couche de capitonnage (4) dans la couche de compression (2), ladite bande de compression étant **caractérisée en ce que** la bande de compression est en outre élastique selon sa direction transversale (T), correspondant à la largeur de la bande de compression.

2. Bande de compression selon la revendication 1, comprenant en outre au moins une couche de mousse (12) s'étendant entre la couche de compression (2) et la couche de capitonnage (4), ladite couche de mousse (12) étant fixée à la couche de compression (2) et à la couche de capitonnage (4) par entremêlement d'une partie des fibres de la couche de capitonnage (4) dans la couche de compression (2) et dans la couche de mousse (12).

3. Bande de compression selon la revendication 2, dans laquelle la couche de mousse (12) présente une épaisseur, mesurée selon une direction perpendiculaire à la direction longitudinale (L) et à la direction transversale (T), sensiblement comprise entre 1 mm et 3,5 mm, mesurée suivant la norme NF EN ISO 5084 :2016, sous une pression de 0,5kPa.

4. Bande de compression selon l'une quelconque des revendications 1 à 3, dans laquelle la couche de compression (2) présente une extensibilité d'au moins 30% selon la direction longitudinale.

5. Bande de compression selon la revendication 4, dans laquelle la couche de compression (2) présente une extensibilité selon sa direction longitudinale sensiblement comprise entre 50% et 120%.

6. Bande de compression selon l'une quelconque des revendications 1 à 5, dans laquelle la couche de compression (2) présente une extensibilité d'au moins 10% selon la direction transversale.

7. Bande de compression selon l'une quelconque des revendications 1 à 6, présentant une masse surfacique sensiblement comprise entre 200 g/m² et 800 g/m².

8. Procédé de réalisation d'une bande de compression (1) selon l'une quelconque des revendications 1 à 7, comprenant les étapes suivantes :
- réaliser un tissu formant une couche de compression (2), ledit tissu étant élastique selon sa direction longitudinale (L) et selon sa direction transversale (T),
- étirer la couche de compression (2) au moins selon sa direction longitudinale (L),
- disposer des fibres sur la couche de compression (2) étirée, ou pré-aiguilleter une nappe de fibres et la disposer sur la couche de compression (2) étirée,
- réaliser une couche de capitonnage (4) et fixer ladite couche de capitonnage (4) sur la couche de compression (2) étirée par entremêlement des fibres entre elles et par entremêlement d'une partie des fibres dans la couche de compression (2).

9. Procédé de réalisation selon la revendication 8 d'une bande de compression (1) selon la revendication 2 ou 3, comprenant une étape de disposition d'au moins une couche de mousse (12) sur la couche de compression (2) étirée, les fibres étant disposées sur la couche de mousse (12), la couche de capitonnage (4) étant réalisée et fixée à la couche de mousse (12) et à la couche de compression (2) par entremêlement des fibres entre elles et par entremêlement d'une partie des fibres dans la couche de mousse (12) et dans la couche de compression (2).

10. Procédé de réalisation selon la revendication 8 ou 9, dans lequel l'étape d'entremêlement des fibres est une étape d'aiguilletage ou d'entremêlement par jet d'air ou jet d'eau.

## Patentansprüche

1. Kompressionsbandage (1) zum Ausüben eines vorbestimmten Drucks auf einen Gliedmaßenabschnitt eines Patienten, die Kompressionsbandage umfassend mindestens eine Kompressionsschicht (2), die dazu bestimmt ist, den vorbestimmten Druck anzuwenden, wobei die Kompressionsschicht mindestens in ihrer Längsrichtung (L) entsprechend der Länge der Kompressionsbandage elastisch ist, und mindestens eine nicht gewebte Polsterschicht (4), die dazu bestimmt ist, an der Haut des Gliedmaßenabschnitts angelegt zu werden, wobei die Polsterschicht (4) durch Verflechten eines Teils der Fasern der Polsterschicht (4) in der Kompressionsschicht (2) befestigt ist, wobei die Kompressionsbandage **dadurch gekennzeichnet, dass** die Kompressionsbandage ferner in ihrer Querrichtung (T) entsprechend der Breite der Kompressionsbandage elastisch ist.

2. Kompressionsbandage nach Anspruch 1, ferner umfassend mindestens eine Schaumstoffschicht (12), die sich zwischen der Kompressionsschicht (2) und der Polsterschicht (4) erstreckt, wobei die Schaumstoffschicht (12) an der Kompressionsschicht (2) und der Polsterschicht (4) durch Verflechten eines Teils der Fasern der Polsterschicht (4) in der Kompressionsschicht (2) und in der Schaumstoffschicht (12) befestigt ist.

3. Kompressionsbandage nach Anspruch 2, wobei die Schaumstoffschicht (12) eine Stärke, gemessen in einer Richtung senkrecht zu der Längsrichtung (L) und zu der Querrichtung (T), von im Wesentlichen zwischen 1 mm und 3,5 mm aufweist, gemessen gemäß der Norm NF EN ISO 5084:2016 unter einem Druck von 0,5 kPa.

4. Kompressionsband nach einem der Ansprüche 1 bis 3, wobei die Kompressionsschicht (2) eine Dehnbarkeit von mindestens 30 % in Längsrichtung aufweist.

5. Kompressionsbandage nach Anspruch 4, wobei die Kompressionsschicht (2) eine Dehnbarkeit in ihrer Längsrichtung im Wesentlichen zwischen 50 % und 120 % aufweist.

6. Kompressionsbandage nach einem der Ansprüche 1 bis 5, wobei die Kompressionsschicht (2) eine Dehnbarkeit von mindestens 10 % in der Querrichtung aufweist.

7. Kompressionsbandage nach einem der Ansprüche 1 bis 6, die ein Flächengewicht von im Wesentlichen zwischen 200 g/m² und 800 g/m² aufweist.

8. Verfahren zur Herstellung einer Kompressionsbandage (1) nach einem der Ansprüche 1 bis 7, umfassend die folgenden Schritte:
- Herstellen eines Gewebes, das eine Kompressionsschicht (2) bildet, wobei das Gewebe in seiner Längsrichtung (L) und in seiner Querrichtung (T) elastisch ist,
- Strecken der Kompressionsschicht (2) zumindest entlang ihrer Längsrichtung (L),
- Anordnen von Fasern auf der gestreckten Kompressionsschicht (2) oder Vornadeln eines Faservlieses und Anordnen auf der gestreckten Kompressionsschicht (2),
- Herstellen einer Polsterschicht (4) und Befestigen der Polsterschicht (4) an der gestreckten Kompressionsschicht (2) durch Verflechten der Fasern untereinander und durch Verflechten eines Teils der Fasern in der Kompressionsschicht (2).

9. Verfahren zur Herstellung nach Anspruch 8 einer Kompressionsbandage (1) nach Anspruch 2 oder 3, umfassend einen Schritt eines Anordnens mindestens einer Schaumstoffschicht (12) auf der gestreckten Kompressionsschicht (2), wobei die Fasern auf der Schaumstoffschicht (12) angeordnet werden, wobei die Polsterschicht (4) hergestellt und an der Schaumstoffschicht (12) und der Kompressionsschicht (2) befestigt wird, indem die Fasern miteinander verflochten werden und indem ein Teil der Fasern in der Schaumstoffschicht (12) und in der Kompressionsschicht (2) miteinander verflochten wird.

10. Herstellungsverfahren nach Anspruch 8 oder 9, wobei der Schritt eines Verflechtens der Fasern ein Schritt eines Vernadelns oder Verflechtens durch Luft- oder Wasserstrahl ist.

## Claims

1. Compression band (1) for exerting a predefined pressure on a limb part of a patient, said compression band comprising at least one compression layer (2) for applying the predefined pressure, said compression layer being elastic at least in its longitudinal direction (L), corresponding to the length of the compression band, and at least one non-woven padding layer (4), for application against the skin of the limb part, said padding layer (4) being secured by intertwining a portion of the fibres of the padding layer (4) in the compression layer (2), said compression band being **characterised in that** the compression band is furthermore elastic along its transverse direction (T), corresponding to the width of the compression band.

2. Compression band according to claim 1, further comprising at least one foam layer (12) extending between the compression layer (2) and the padding layer (4), said foam layer (12) being attached to the compression layer (2) and the padding layer (4) by entangling a portion of the fibres of the padding layer (4) in the compression layer (2) and in the foam layer (12).

3. Compression band according to claim 2, wherein the foam layer (12) has a thickness, measured in a direction perpendicular to the longitudinal direction (L) and the transverse direction (T), substantially between 1 mm and 3.5 mm, measured according to NF EN ISO 5084:2016, under a pressure of 0.5 kPa.

4. Compression band according to any one of claims 1 to 3, wherein the compression layer (2) has an extensibility of at least 30% in the longitudinal direction.

5. Compression band according to claim 4, wherein the compression layer (2) has an extensibility in its longitudinal direction of substantially 50% to 120%.

6. Compression band according to any one of claims 1 to 5, wherein the compression layer (2) has an extensibility of at least 10% in the transverse direction.

7. Compression band according to any one of claims 1 to 6, having a basis weight substantially 200 g/m² to 800 g/m².

8. Method of manufacturing a trim element according to any one of claims 1 to 7, comprising the following steps:
- making a fabric forming a compression layer (2), said fabric being elastic in its longitudinal direction (L) and in its transverse direction (T),
- stretching the compression layer (2) at least in its longitudinal direction (L),
- laying out fibres on the stretched compression layer (2), or pre-needling a fibre web and laying it out on the stretched compression layer (2),
- making a padding layer (4) and attaching said padding layer (4) to the stretched compression layer (2) by entangling the fibres with each other and by entangling part of the fibres in the compression layer (2).

9. Method of making according to claim 8 a compression band (1) according to claim 2 or 3, comprising a step of arranging at least one layer of foam (12) on the stretched compression layer (2), the fibres being arranged on the foam layer (12), the padding layer (4) being produced and fixed to the foam layer (12) and to the compression layer (2) by intertwining the fibres with one another and by intertwining a part of the fibres in the foam layer (12) and in the compression layer (2).

10. A method of making according to claim 8 or 9, wherein the fibre intertwining step is one of needling or intertwining by air jet or water jet.
